# EUROPEAN PATENT APPLICATION

(11) **EP 4 837 615 A2**
(43) Date of publication of application: **11.11.2026**
(21) Application number: 26193408.7
(22) Date of filing: 29.05.2019
(51) Int. Cl.: A61M 35/00

(54) **DRUG DELIVERY METHODS AND SYSTEMS**

(30) Priority: 29.05.2018 US 201862677494 P
(62) Document number(s) of the earlier application(s) in accordance with Art. 76 EPC: 19812316.8 / 3 801 732
(71) Applicant: Morningside Venture Investments Limited, 98000 Monaco (MC)
(72) Inventor: TONG, Ling-Kang, Hayward, 94545 (US); BUCHMAN, Daniela Tamar, Hayward, 94545 (US); HANCOCK, Jackie Joe, Hayward, 94545 (US); TERZULLI, Nicholas, Hayward, 94545 (US)
(74) Representative: Grünecker Patent- und Rechtsanwälte PartG mbB

(57) **Abstract**

A two-part bioactive agent delivery system includes a disposable part, a reusable part, and a solvent removal element. The disposable part includes an agent reservoir, a transdermal patch communicating with the agent reservoir and adapted to transdermally deliver the bioactive agent to a user. The transdermal patch has a bottom surface adapted to contact skin of the user, a top surface opposite the bottom surface, and a gas permeable membrane disposed over the top surface of the transdermal patch. The reusable part includes a power source and control electronics that are adapted to deliver bioactive agent dissolved in a solvent from the agent reservoir to the transdermal patch. The solvent removal element includes a gap disposed between the disposable part and the reusable part to create a flow path for gaseous solvent to flow from the gas permeable membrane to ambient air around the bioactive agent delivery system.

## Description

### CROSS-REFERENCE TO RELATED APPLICATIONS

This application claims priority to U.S. Provisional Patent Application No. 62/677,494, filed on May 29, 2018, the entirety of which is incorporated by reference herein.

This application may also be related to U.S. Patent Application No 15/699,382, filed on September 8, 2017, the entirety of which is incorporated by reference herein.

### STATEMENT AS TO FEDERALLY SPONSORED RESEARCH

This invention was made with Government support under Contract No. 2R22CA171786-04 awarded by the National Institute of Health. The Government has certain rights in the invention.

### INCORPORATION BY REFERENCE

All publications and patent applications mentioned in this specification are herein incorporated by reference in their entirety to the same extent as if each individual publication or patent application was specifically and individually indicated to be incorporated by reference.

### BACKGROUND

Medicinal drugs are given to people to manage or improve their health for a variety of reasons, such as to prevent or treat a medical condition or disease such as diabetes, Parkinson's disease, ulcerative colitis, or to manage nicotine or another addiction or dependency, or to manage pain.

Some medicinal drugs are rapidly metabolized by the body. As a result, multiple doses of the drug over a period of time are often needed to provide a desired effect. In addition to having desired preventative or therapeutic effects, medicinal drugs can also have negative side-effects on the body that can range from irritating to life-threatening. Further, a person's body can develop tolerance to a drug, experience a diminished response to the drug after taking it for a period of time, and require higher doses to have an effect, resulting in increased drug use and additional side-effects. It is therefore beneficial to a person taking a drug to minimize the amount of drug he or she takes to prevent or minimize tolerance and other unwanted side-effects while still receiving the desired therapeutic effect from the drug.

Tobacco use (such as smoking) causes serious health problems and can lead to premature death. According to the United States Center for Disease Control (CDC), tobacco use causes more than 5 million deaths per year and contributes to the development of serious illnesses such as cancer, diabetes, heart disease, lung disease (bronchitis, chronic airway destruction, emphysema), and stroke. Despite anti-smoking advertising campaigns, legislation, taxation, and development of smoking cessation products to stop or prevent people from using tobacco, tobacco sales remains a multibillion dollar industry, generating an estimated $35 billion dollars per year in profits. Tobacco initially causes physical and mood-altering effects that are temporarily pleasing. Further, it is difficult for a person to stop using a tobacco product because tobacco contains nicotine. Nicotine is highly addictive, and not having the nicotine causes harsh withdrawal symptoms. It is very difficult for a person to overcome a nicotine addiction and stop smoking.

Medicinal drugs can be taken by tobacco users to help them overcome their nicotine addiction. Some products to help a person stop smoking contain small amounts of nicotine as a medicinal drug to minimize withdrawal symptoms and gradually wean a person from their nicotine addiction. Medicinal smoking cessation drugs such as nicotine have to be taken over an extended period of time (often over the course of many months) to give the body time to adjust to having less nicotine. Medicinal drugs, medical devices, and other products, including smoking cessation products, are regulated in the United States by the U.S. Food and Drug Administration (FDA). FDA approved products on the market to help a person quit smoking include various medicinal drugs that require a doctor's prescription as well as over-the-counter products. These products include capsules or tablets, gums, inhalers, lozenges, nasal sprays, and skin patches. These products have thus far been inadequate to get people to stop smoking: 68.9 % of adult cigarette smokers say they want to stop smoking, and every year some 42.7% make an attempt to stop smoking, but are unsuccessful.

Existing smoking cessation products and other therapeutic and prophylactic treatments for health issues suffer from a variety of problems. They may be inconvenient or socially awkward to use. They may require careful and troublesome tracking of when they were used and how much was used to prevent overdosing. They may act too slowly after being administered and not produce a desired effect when it's needed. They may not be readily available when needed (such as while a person is sleeping). None have been wholly effective to for preventing or treating various medical or other conditions, and smoking remains a significant health and social problem.

Thus, new and improved drug delivery systems for bioactive agents, such as smoking cessation agents, are needed.

### SUMMARY OF THE DISCLOSURE

In general, in one embodiment, a two-part bioactive agent delivery system includes a disposable part, a reusable part, and a solvent removal element. The disposable part includes an agent reservoir, a transdermal patch communicating with the agent reservoir and adapted to transdermally deliver the bioactive agent to a user. The transdermal patch has a bottom surface adapted to contact skin of the user, a top surface opposite the bottom surface, and a gas permeable membrane disposed over the top surface of the transdermal patch. The reusable part includes a power source and control electronics that are adapted to deliver bioactive agent dissolved in a solvent from the agent reservoir to the transdermal patch. The solvent removal element includes a gap disposed between the disposable part and the reusable part to create a flow path for gaseous solvent to flow from the gas permeable membrane to ambient air around the bioactive agent delivery system.

This and any other embodiments can include one or more of the following elements. The gap can extend between parallel features on the disposable part and the reusable part. The gap can be formed when the disposable part and the reusable part are releasably engaged. The gap can be maintained with a spacer disposed between the disposable part and the reusable part. The spacer can extend from the disposable part. The spacer can extend from the reusable part. The system can further include at least one drainage port formed in an exterior surface of the reusable part or the disposable part. The disposable part can further include an agent outlet communicating with the agent reservoir and the transdermal patch. The agent outlet can be configured to provide the bioactive agent dissolved in the solvent to a space between the transdermal patch and the vapor permeable membrane. The agent reservoir can include a piston movably disposed in a chamber. The system can further include a spring extending between the agent reservoir piston and a surface to pressurize the agent reservoir when the spring is compressed and the agent reservoir contains a quantity of bioactive agent solution. The system can further include a bolus chamber communicating with the agent reservoir and an agent outlet, and the bolus chamber can include a piston movably disposed in a chamber. The volume of the bolus chamber can be less than the volume of the agent reservoir. The system can further include a valve having a first position communicating the agent reservoir with the bolus chamber and a second position communicating the bolus chamber with the agent outlet. The reusable part can include a valve driver, and the control electronics can be adapted to control the valve driver to actuate the valve to deliver bioactive agent solution from the agent reservoir to the bolus chamber and from the bolus chamber to the agent outlet. The system can further include a spring extending between the bolus chamber piston and a surface to pressurize the bolus chamber when the spring is compressed and the bolus chamber contains a quantity of bioactive agent solution. The system can further include a latch adapted to removably attach the disposable part to the reusable part. The latch can be positioned on one side of the system and a rail can be positioned along a second side of the system. The disposable part and the reusable part can be configured to slide relative to one another along the rail until the latch is activated to attach the disposable part to the reusable part. The system can further include a connection detector adapted to detect a connection between the disposable part and the reusable part. The connection detector can include a magnet. The magnet can be disposed in the disposable part. The magnet can be disposed in the reusable part. The connection detector further can include a magnetoresistive switch.

In general, in one embodiment, a method of delivering bioactive agent includes: connecting a reusable part of a delivery system to a disposable part of the delivery system to form a flow path between the reusable part and the disposable part, delivering the bioactive agent dissolved in a solvent from a reservoir of the disposable part to a transdermal membrane of the disposable part, and allowing the solvent to evaporate and flow from the transdermal membrane through a gas permeable membrane and along the flow path to ambient air around the delivery system.

This and any other embodiments can include one or more of the following elements. The method can further include applying the transdermal membrane to skin of a user. The method can further include delivering the bioactive agent to the skin. The delivering step can include controlling with the reusable part movement of the bioactive agent dissolved in the solvent from the reservoir to the transdermal membrane. The controlling step can include actuating a valve. The method can further include detecting a connection between the disposable part and the reusable part. The detecting step can include sensing a magnetic field.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
Figure 1 is a perspective view of a two-part bioactive agent delivery system according to an embodiment.
Figure 2 is a perspective view of a partially connected two-part bioactive agent delivery system according to an embodiment.
Figure 3 is a perspective view of a portion of the disposable part of the two-part bioactive agent delivery system of Figure 1 or Figure 2.
Figure 4 is an exploded view of the disposable part of the two-part bioactive agent delivery system of Figure 1 or Figure 2.
Figure 5 is a side view of a chassis of the disposable part of the two-part bioactive agent delivery system of Figure 1 or Figure 2.
Figure 6 is a side view of the two-part bioactive agent delivery system of Figure 1 or Figure 2.
Figure 7 is an exploded view of the reusable part of the two-part bioactive agent delivery system of Figure 1 or Figure 2.
Figure 8 shows a connection sensor for a two-part bioactive agent delivery system according to an embodiment.
Figure 9 is a perspective view of a partially connected two-part bioactive agent delivery system according to an embodiment.
Figure 10 is a perspective view of the two-part bioactive agent delivery system of Figure 9.
Figure 11 is a top view of the disposable part of the two-part bioactive agent delivery system of Figure 9.
Figure 12 is a top view of the reusable part of the two-part bioactive agent delivery system of Figure 9.
Figure 13 is a top view of the two-part bioactive agent delivery system of Figure 9.
Figures 14-16 are additional perspective views of the partially connected two-part bioactive agent delivery system of Figure 9.
Figure 17 is another perspective view of the two-part bioactive agent delivery system of Figure 9.
Figure 18 is a top view of the chassis of the two-part bioactive agent delivery system of Figure 9.
Figures 19A-19C are side view of the two-part bioactive agent delivery system of Figure 9.
Figures 20A-20C are perspective views of the disposable part of the two-part bioactive agent delivery system of Figure 9.
Figure 21A is another perspective view of the disposable part of the two-part bioactive delivery system of Figure 9.
Figure 21B is an exploded view of the disposable part of Figure 21A.
Figure 21C is an exploded view of the fluidics assembly of Figures 21A and 21B.
Figure 22 is an exploded view of the reusable part of the two-part bioactive agent delivery system of Figure 9.

### DETAILED DESCRIPTION

Described herein are systems and methods for delivering bioactive agents. The systems can include two parts: a disposable part and a reusable part. The disposable part may include bioactive agent and system parts that come into contact with bioactive agent, and the reusable part may not contain bioactive agent and system parts that come into contact with bioactive agent.

In some embodiments, the disposable part can include an agent reservoir, a bolus chamber, a valve alternately communicating the reservoir with the bolus chamber and the bolus chamber with an outlet, a solvent removal element, and a transdermal membrane receiving the agent from the outlet. The reusable part can include a valve driver and control electronics.

These bioactive agent delivery systems described herein can deliver a solution of the bioactive agent to a transdermal patch. Solvent from the bioactive agent solution can evaporate from the transdermal patch through the solvent removal element (e.g., a gap between the reusable part and the disposable part) to control the manner in which the bioactive agent passes from the patch into the user's skin.

A bioactive agent delivery system as described herein may be useful for delivering a bioactive agent to any part on or in a user's body. In some particular variations, a bioactive agent delivery system as described herein may be especially useful for delivering a bioactive agent topically or transdermally to or through a user's skin to a skin layer or bloodstream. Effective topical or transdermally delivery may be aided by use of a skin delivery membrane such as described herein for transferring active agent across the skin that is fully or sufficiently wetted to effectively transfer a dose of bioactive agent to a user's skin. In some variations, a bioactive agent delivery system may be electronically controlled, programmable, portable, and/or wearable.

The bioactive agent delivery systems described herein may reproducibly deliver a fixed amount of a bioactive agent to a user, such as to a user's skin, to have a therapeutic or prophylactic effect on the user. In some variations, the bioactive agent delivery systems may be configured to be wearable and to deliver a fixed amount of a bioactive agent to a user's skin in a relatively thin, quiet, easy to use, convenient, electronically controlled system. The bioactive agent delivery systems may be configured to be attached to a user's body (such as for a day or shorter or longer), connect with a user's skin, and deliver a bioactive agent across the user's skin.

The bioactive agent delivery systems as described herein may be useful for delivering a bioactive agent to a user for addiction or dependency management or prevention such as for a drug addiction, for diabetes or other disease management or prevention, for pain management or prevention, or for another therapeutic or prophylactic purpose. The systems, devices, and methods may be especially useful for delivering multiple doses of a drug or other bioactive agent to a user over time with a safe, inexpensive, convenient, and easy-to-use system that minimizes risk of a drug or other bioactive agent overdose.

A bioactive agent delivery system as described herein may be configured to deliver multiple doses or boluses during the course of a day and/or for multiple days. Delivering multiple boluses may be especially useful to help a user control cravings or other withdrawal symptoms by delivering a bioactive agent dose (e.g., a dose of nicotine) especially during (or before) a time of day when cravings or withdrawal symptoms are normally most troublesome (such as delivering a dose during the night to prevent cravings upon waking).

Further, having a bioactive agent delivery system with a disposable part may allow the system to be relatively small or flat and easy to wear. For example, a system may be relatively small or flat because the disposable part needs only contain a limited amount of bioactive agent and/or the reusable part needs only be imparted with sufficient force or power for a limited number of dose deliveries before being recharged. In some examples, a bioactive delivery system as described herein may be less than about 20 mm, less than about 16 mm, less than about 15 mm, less than about 14 mm, less than about 13 mm, less than about 12 mm, less than about 11 mm or less than about 10mm in thickness and may be less than 40 mm, less than 35 mm in length or width, or less than 30 mm in length or width. In some examples, a system may have less than 1500 mm² or less than 1000 mm² top (or bottom) surface area. "Bottom" in this context generally refers the part(s) of the system closest to a user. If a system includes a transdermal patch, bottom may refer to the transdermal patch and to the skin delivery member of a transdermal patch. A surface area of one side of a skin delivery member may be at least 100 mm2, at least 500 mm2, at least 1500 mm2, at least 2000 mm2, at least 2500 mm2 at least 3000 mm2 or less than or between any of these numbers (such as at least 500 mm2 and less than 2000 mm2).

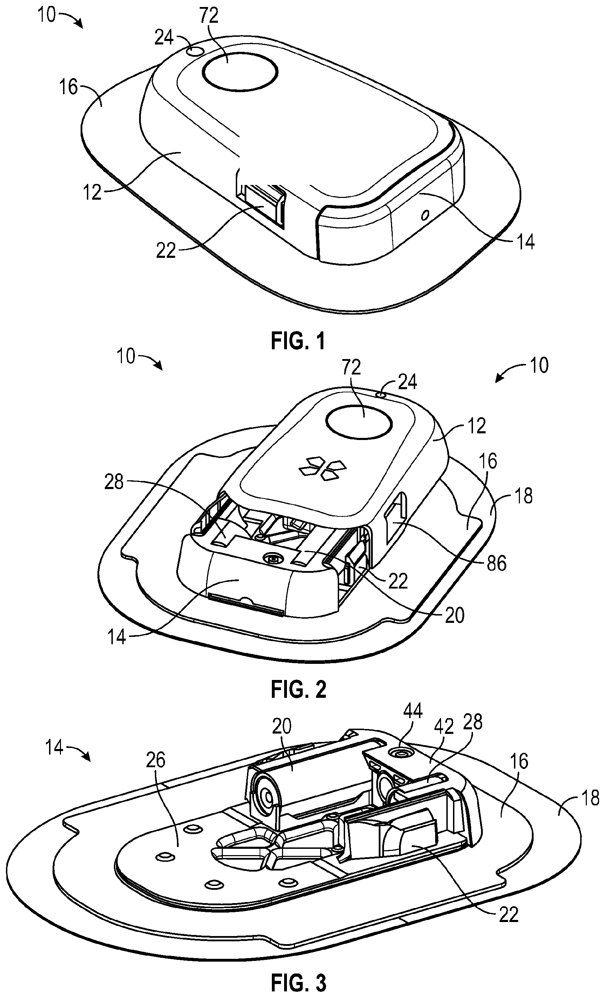

Figures 1 and 2 show a two-part bioactive agent delivery system according to an embodiment of this invention. The system 10 has a reusable part 12 connectable to a disposable part 14. An adhesive element 16 (such as, e.g., adhesive foam) extends around the periphery of the disposable part 14, and a removable release liner 18 covers the adhesive surface of the adhesive element 16. The disposable part 14 also has a reservoir 20 containing a solution of the bioactive agent (e.g., nicotine) to be delivered transdermally to user. The reusable part 12 has control electronics, a user interface button 72, and an LED mode status indicator 24. One or more latches 22 may be used to connect and disconnect the disposable part 14 from the reusable part 12. An outer surface of the bioactive agent delivery system 10 when the reusable part 12 and the disposable part 14 are connected together may have any shape, such as circular, ovoid, rectangular, square, and may be contoured (or able to be contoured) to better fit a user's skin

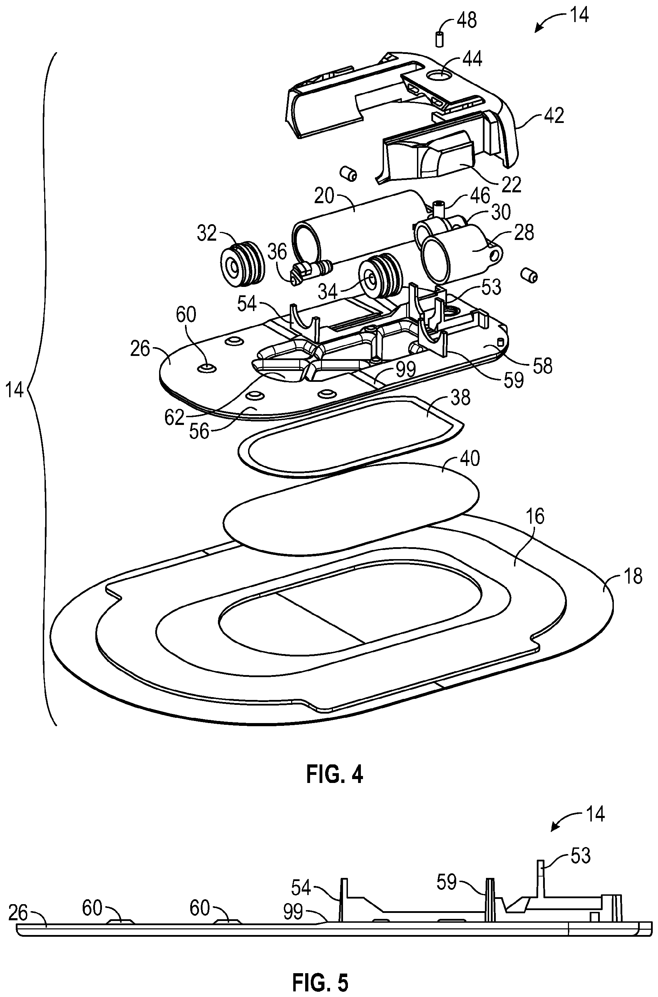

Further details of the disposable part 14 are shown in Figures 3-5. Mounted on a chassis 26 are the reservoir 20, a bolus chamber 28, and a valve chamber 30. A manifold communicates the reservoir 20 with the valve chamber 30 and the valve chamber 30 with the bolus chamber 28. Movable pistons 32 and 34 are disposed in the reservoir 20 and bolus chamber 28, respectively. A rotatable valve element 36 is disposed in valve chamber 30.

Attached to the underside of chassis 26 (e.g., by heat bonding) are a gas permeable membrane 38 (formed, e.g., from Poreflon^{®} polytetrafluoroethylene) and a transdermal drug delivery patch 40 (e.g., a Celgard^{®} membrane). An upper housing 42 supports latch(es) 22 and partially covers reservoir 20, valve chamber 30, bolus chamber 28, and chassis 26. An opening 44 in housing 42 leads to a fill port 46 that can be used to add a solution of the bioactive agent to reservoir 20 and bolus chamber 28. A fill port plug 48 seals fill port 46 after filling the reservoir and bolus chamber with a solution of the bioactive agent.

Chassis 26 has supports 53, 54, and 59 engaging the reservoir 20, valve chamber 30, and bolus chamber 28, respectively, to hold the reservoir 20, valve chamber 30, and bolus chamber 28 in place. One or more raised spacers 60 extend upwardly from the top side of chassis 26. Open vents 62 in chassis 26 are disposed over the gas permeable membrane 38. Spacers 60 engage the underside of the reusable part 12 to establish a gap 64 forming a flow path for evaporated solvent passing from transdermal patch 40 through gas permeable membrane 38 and chassis vents 62, as described below. In an alternative embodiment, the one or more spacers 60 may extend from the reusable part instead of, or in addition to, the spacer(s) extending from the disposable part. Ramped ledges 99 can transition the chassis from a thinner section 56 to a thicker section 58.

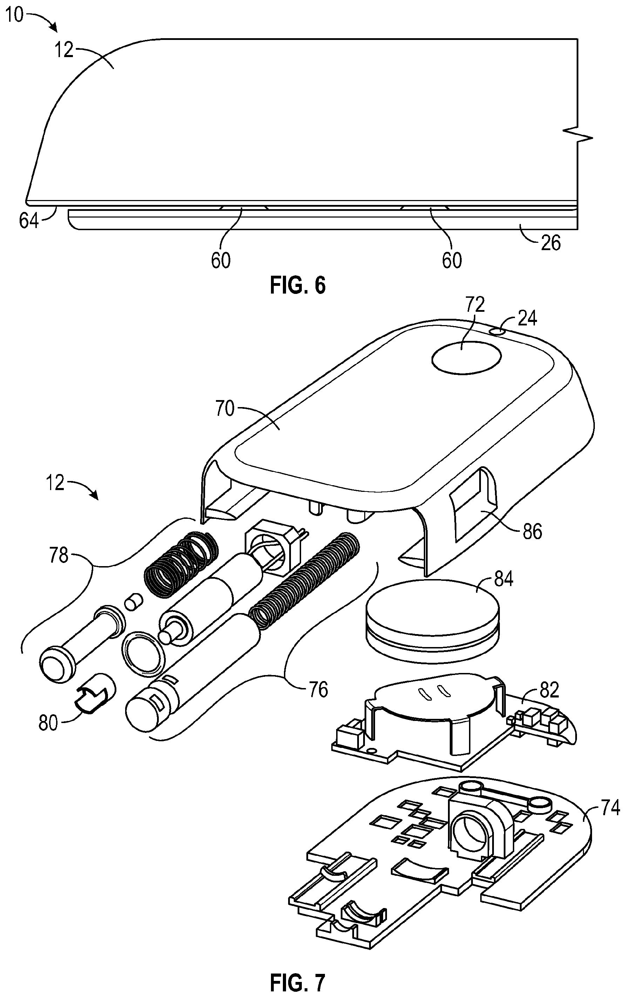

Figure 7 shows components of the reusable part 12. A latch element 86 adapted to engage with the latch 22 of the disposable part is formed in an upper housing 70. Disposed between an upper housing 70 and a lower housing 74 are a spring assembly 76 for the reservoir 20 and a spring assembly 78 for the bolus chamber 28. When the disposable part 14 is engaged and latched with the reusable part 12, the spring assemblies 76 and 78 engage with the pistons 32 and 34, respectively, and the springs of the spring assemblies 76, 78 compress to pressurize the bioactive agent solution in reservoir 20 and bolus chamber 28, respectively. A motor assembly 80 engages valve element 36 and operates under the control of a microprocessor on the printed circuit board 82 using power from battery 84 to turn valve element 36 between a position in which reservoir 20 communicates with bolus chamber 28 and a position in which bolus chamber 28 communicates with patch 40. A control button 72 in the upper housing 70 communicates with the microprocessor. The control button 72 can further serve as a user input mechanism. It may be depressed, e.g., to power on the electronics; to pair the device with a smartphone application; to provide information about the user, such as the experience of a craving for a cigarette; or to request a device status update or other information. Status indicator 24 can indicate the status of the system, e.g., whether the system is delivering fluid, in stand-by mode, or empty of bioactive agent.

The system 10 may be used to deliver a bioactive agent transdermally to a user. Disposable part 14 may be prefilled with a solution of the bioactive agent. The disposable part 14 may be connected to the reusable part 12 and latched with latch components 22 and 86. This connection compresses spring assemblies 76 and 78 against pistons 32 and 34 of reservoir 20 and bolus chamber 28, respectively, to pressurize the bioactive agent solution in the reservoir 20 and bolus chamber 28. Release liner 18 may be removed so that the adhesive on the underside of adhesive element 16 can be attached to the user's skin. In response to a signal from the device's microprocessor, motor assembly 80 turns rotatable valve element 36 to a position in which the contents of bolus chamber 28 are delivered through an agent outlet in chassis 26 to a space between gas permeable membrane 38 and transdermal patch 40. As the bioactive agent moves from the patch 40 into the user's skin, solvent from the bioactive agent solution evaporates and passes through gas permeable membrane 38 and the vents 62 in chassis 26 to reach the flow path defined by gap 64. Removal of solvent from the transdermal patch 40 enables the percentage of bioactive agent in liquid solution in the patch to remain high enough to maintain a desired transdermal delivery rate of the bioactive agent until essentially all of the bioactive agent in the bolus of bioactive agent solution has been delivered. Subsequent boluses of bioactive agent may be delivered by actuating the valve element 36 to enable refilling of the bolus chamber 28 with solution from the reservoir 20 followed by movement of the valve element 36 to permit delivery of the next bolus from bolus chamber 28 to the transdermal patch 40. The timing of the bolus deliveries are under the control of the programmed microprocessor.

Further details of the operation of the reusable and disposable parts and components, including reservoir 20, bolus chamber 28, and valve 36 and/or the control button 72 and indicator 24, to deliver the bioactive agent solution to the transdermal patch at controlled times may be found in US Publication No. 2016/0220798, the entirety of which is incorporated by reference.

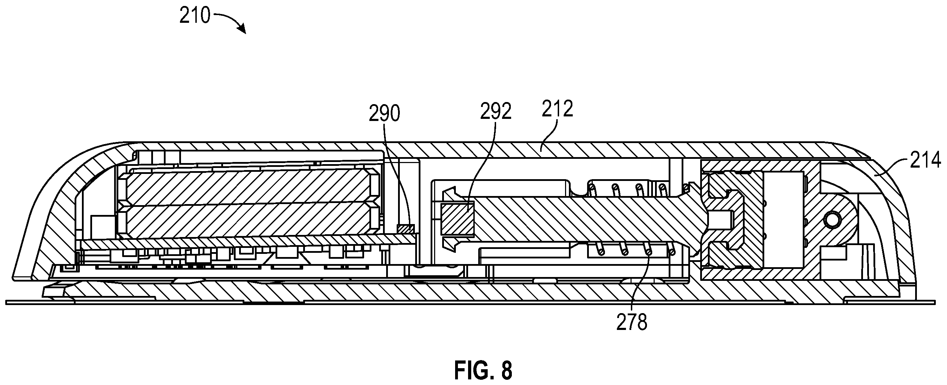

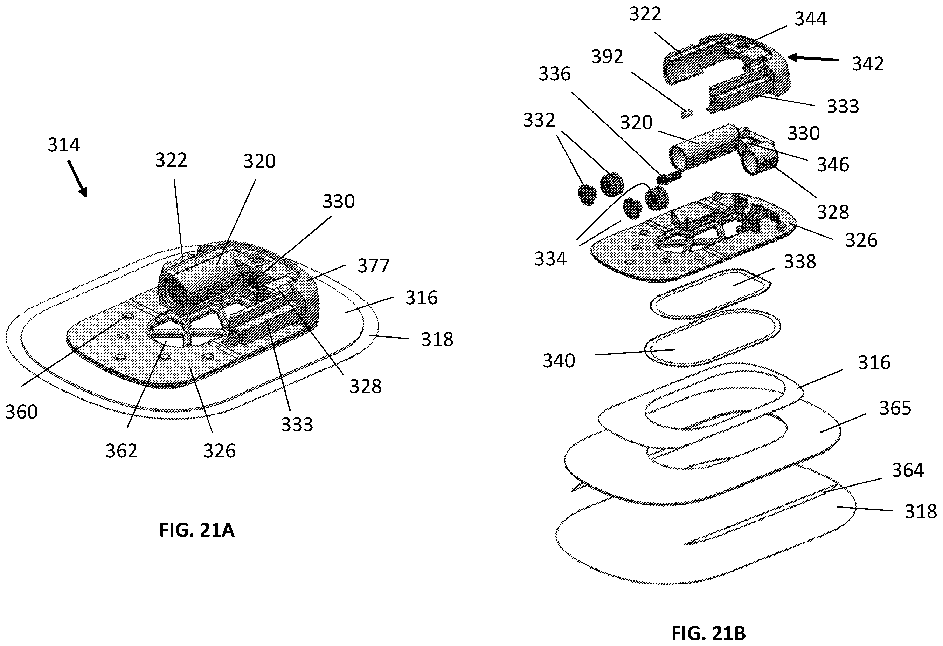

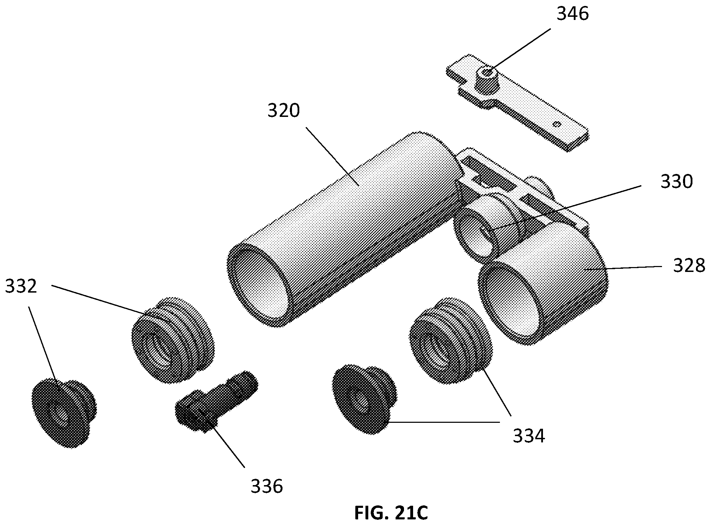

In some embodiments, the systems described herein can include a sensor to detect connection of a disposable part with the reusable part. In one embodiment shown in Figure 8, a system 210 includes a magnetoresistive switch 290 disposed in the reusable part 212 that detects a magnet 292 disposed in the spring assembly 278. In an alternative embodiment, the magnet can be disposed in the disposable part 214 (see, for example, magnet 392 in Figure 21B). Use of the magnetoresistive switch 290 enables the electronics to be encapsulated and waterproof. In some embodiments, a reed switch or a Hall sensor may be used as an alternative to the magnetoresistive switch. Information about the connection times and durations of the reusable part with the disposable part may be used, e.g., to monitor compliance with a drug delivery regimen.

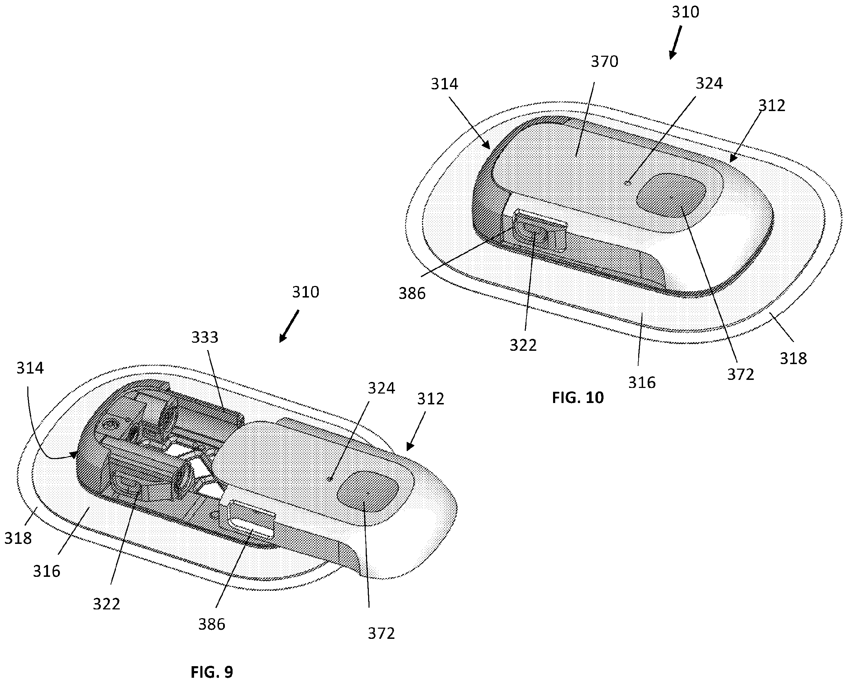

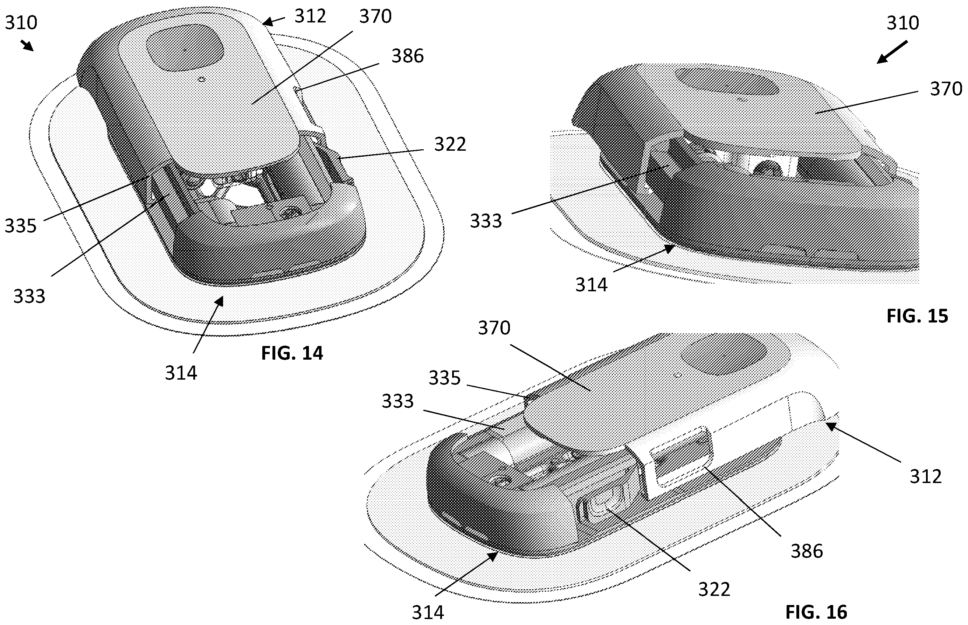

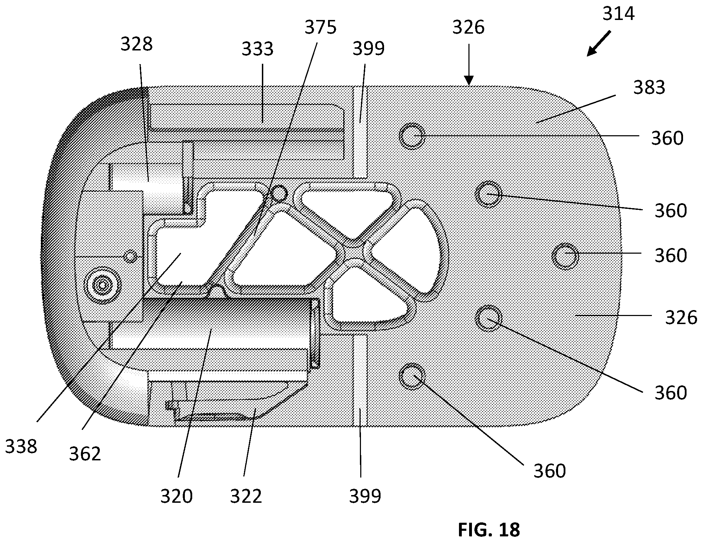

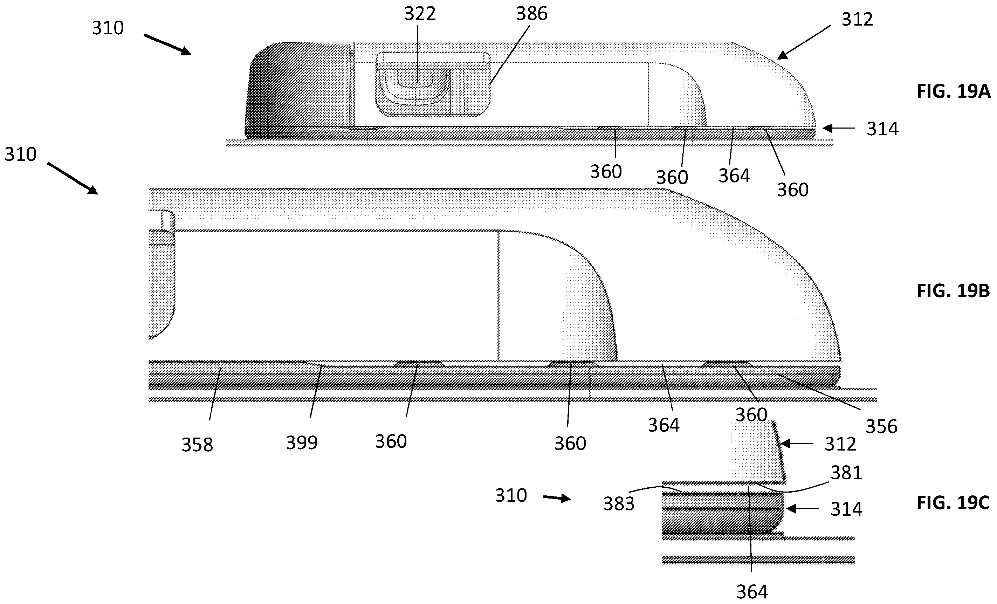

Another exemplary two-part bioactive agent delivery system 310 that is similar to system 10 is shown in Figures 9-22. Referring to Figures 9-10, system 310 includes a reusable part 312 connectable to a disposable part 314, an adhesive element 316, and release liner 318. The reusable part 312 has control electronics, a user interface button 372, and an LED mode status indicator 324. When the reusable part 312 and the disposable part 314 are connected together, the upper housing 342 of the disposable part 314 and the housing 370 of the reusable part abut one another to create a substantially smooth outer contour.

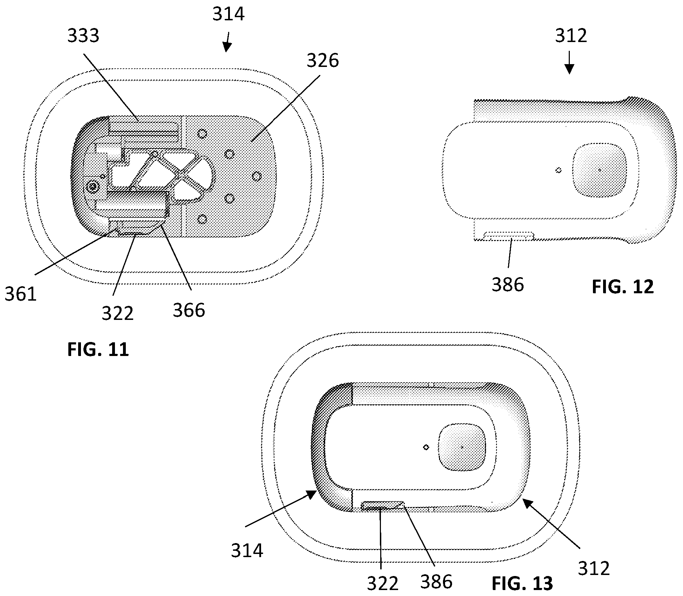

As shown in Figures 11-16, the system 310 further includes a single latch 322 and rail 333 to connect and disconnect the disposable part 314 from the reusable part 312. The latch 322 can be on a first side of the disposable part 314 while the rail 333 can be positioned on a second opposite side of the disposable part 314. The rail 333 can extend substantially parallel with the longitudinal axis of the system 310. Further, the rail 333 can be configured to mate with a sliding element 335 (e.g., a c-shaped element) on the housing 730 of the reusable part 312. In use, the sliding element 335 can slide along the rail 333 as the disposable part 314 and reusable part 312 are engaged. The rail 333 can thus advantageously ensure that the disposable part 314 and reusable part 312 remain aligned as they are connected or disconnected. The latch 322 can have a cantilevered first end 361 and a sloped or ramped second end 366 (see Figure 11). The ramped second end 366 can be connected to the upper housing 342 of the disposable part 314 while the cantilevered first end 361 can be configured to mate with a latch element 386 (e.g., an opening) on the housing 370 of the reusable part 312. The latch 322 and latch element 386 can function to hold the disposable part 314 and reusable part 312 together until the latch 322 is depressed by the user.

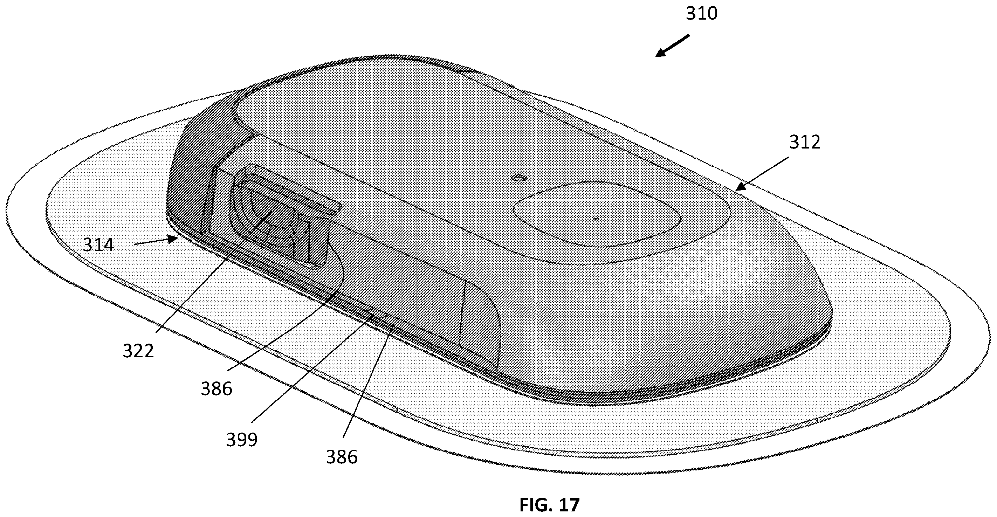

Referring to Figures 17-19C, the system 310 can include open vents 362 through the chassis 326 so as to expose the membrane 338 thereunder. The vents 362 can be positioned in a central portion of the chassis 326 and can be cut so as to maximize the exposure of the surface of the membrane 338. For example, the vents 362 can expose 5-30% of the surface of the membrane 338, such as 10-20% of the surface. Diagonal struts 375 can extend between the vents 362 to maintain the strength of the chassis 362. The vents 326 can connect to gap 364 between the reusable part 312 and the disposable part 314. The gap 364 can be formed between the parallel bottom surface 381 of the reusable part 312 and top surface 383 of the disposable part 314. The gap 364 can be formed by the framework of the system, including the mating between the rail 333 and sliding element 335 and the mating between the latch 322 and latch element 382. Additionally, one or more spacers 360 can be configured to help maintain the gap 364. The spacers 360 can be, for example, cylindrical or semi-spherical elements that protrude upwards (i.e., from the top surface 383 of) the chassis 326. Further, the spacers 360 can be positioned on a side of the chassis 360 that is opposite to the reservoir 320, bolus chamber 320, and valve chamber 330 (e.g., on the thinner section 356). There can be a plurality of spacers 360, such as 3-5 spacers 360, that are substantially equally spaced around the vents 362. Each of the spacers 360 can have a small diameter (e.g., a diameter that is less than 10% of the width of the chassis 26). Additionally, ramped ledges 399 can transition between the thinner section 356 of the chassis 326 and the thicker section 358 of the chassis 326. In some embodiments, there can be two ledges 399 that are axially aligned with one another at approximately the center of the chassis 326.

The spacers 360 can engage the bottom surface 381 of the reusable part 312 to maintain the gap 364 and therefore to form a flow path for evaporated solvent passing through the vents 362. In an alternative embodiment, the one or more spacers may extend from the reusable part instead of, or in addition to, the spacers extending from the disposable part. Additionally, the thick section 358 of the chassis 326 can engage the bottom surface 381 of the reusable part 312 (i.e., can be coincident with the bottom of the reusable part 312), which can further maintain the gap 364 above the thin section 356.

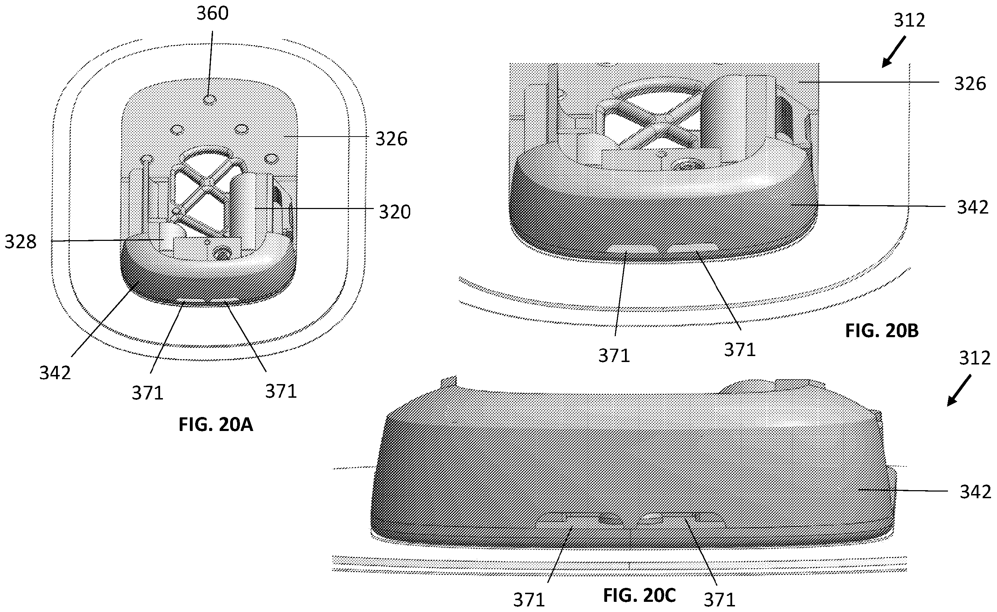

Referring to Figures 20A-20C, the system 310 can further include one or more drainage ports 371 therein that are aligned with or connected with the gap 364 and can provide drainage for any excess fluid left in the side of the system 310. The drainage ports 371 can be formed in the upper housing 377 of the disposable part 312 proximate to the thicker section 358 of the chassis 326 and can facilitate water drainage (e.g., from the user bathing and/or otherwise getting fluid in or on the system 310). In some embodiments, the drainage ports 371 can be positioned such that, when the system is worn on the body (e.g., on the arm) and the device 310 is turned vertically, water will exit the device by gravity via ports 371. The ports 371 can be sized so as to avoid capillary pooling of water therein.

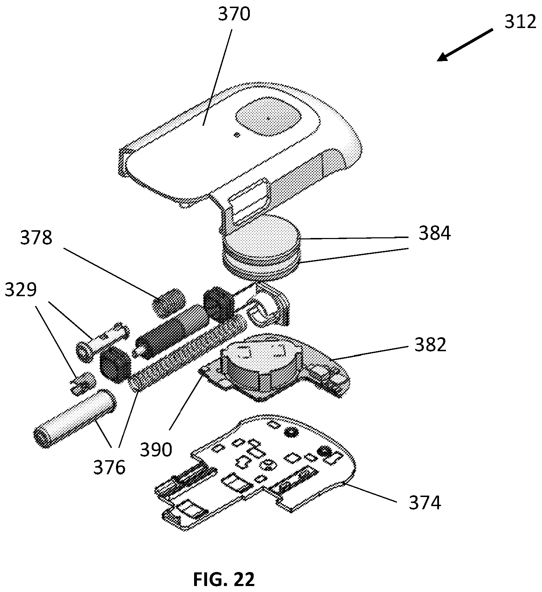

Further details of the disposable part 314 are shown in Figures 21A-21C, and further details of the reusable part 312 are shown in Figure 22. The disposable part 314 can further include a transdermal delivery patch 340, a permeable membrane 338 and a foam adhesive 365 with deadening strips 364. The deadening strips 364 can provide the user with an edge or small flap to grab onto to help remove the system 310 from the skin. An opening 344 in housing 342 leads to a fill port 346 that can be used to add a solution of the bioactive agent to reservoir 320 and bolus chamber 328. Movable pistons 332 and 334 are disposed within the reservoir 320 and bolus chamber 328, respectively. The reusable part 312 can include a printed circuit board 382 positioned over the lower housing 374, bolus spring assembly 378 (to control piston 334), reservoir spring assembly 376 (to control piston 332). A motor 329 can control activation of the valve 336 and spring assemblies 376, 378. A power supply 384 (e.g., batteries) can provide power to the motor 329. A detect magnet 392 can be positioned in the disposable part 314 while a magnetoresistive switch 390 can be positioned on the printed circuit board 382 of the reusable part 312 to detect attachment of the disposable part 314 to the reusable part 312 (i.e., when the magnet 392 and switch 390 are adjacent to one another upon attachment of the reusable part 312 and the disposable part 314).

The system 310 can operate similar to system 10. The disposable part 314 may be connected to the reusable part 312 by sliding the rail 333 and sliding element 382 relative to one another until the components are latched with latch components 322 and 386. This connection compresses spring assemblies 376 and 378 against pistons 332 and 334 of reservoir 320 and bolus chamber 328, respectively, to pressurize the bioactive agent solution in the reservoir 320 and bolus chamber 328. In response to a signal from the device's microprocessor, motor assembly 380 turns rotatable valve element 336 to a position in which the contents of bolus chamber 328 are delivered to the patient's skin through the transdermal delivery patch 340. As the bioactive agent moves onto the user's skin, solvent from the bioactive agent solution evaporates through the gas permeable membrane 338 and the vents 362 in chassis 326 to reach the flow path defined by gap 364. Further details of the operation of the reusable and disposable parts and components to deliver the bioactive agent solution to the transdermal patch at controlled times may be found in US Publication No. 2016/0220798, the disclosure of which is incorporated by reference.

It should be understood that any element described herein with respect to one embodiment can be added to or substituted for any element described with respect to another embodiment.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.
Further disclosed are the following items:
1. A two-part bioactive agent delivery system, the system comprising:
   a disposable part comprising
      an agent reservoir,
      a transdermal patch communicating with the agent reservoir and adapted to transdermally deliver the bioactive agent to a user, the transdermal patch having a bottom surface adapted to contact skin of the user and a top surface opposite the bottom surface, and
      a gas permeable membrane disposed over the top surface of the transdermal patch;
   a reusable part comprising a power source and control electronics, the control electronics being adapted to deliver bioactive agent dissolved in a solvent from the agent reservoir to the transdermal patch; and
   a solvent removal element comprising a gap disposed between the disposable part and the reusable part to create a flow path for gaseous solvent to flow from the gas permeable membrane to ambient air around the bioactive agent delivery system.
2. The system of item 1, wherein the gap extends between parallel features on the disposable part and the reusable part.
3. The system of item 1, wherein the gap is formed when the disposable part and the reusable part are releasably engaged.
4. The system of item 1, wherein the gap is maintained with a spacer disposed between the disposable part and the reusable part.
5. The system of item 4, wherein the spacer extends from the disposable part.
6. The system of item 4, wherein the spacer extends from the reusable part.
7. The system of item 1, further comprising at least one drainage port formed in an exterior surface of the reusable part or the disposable part.
8. The system of item 1, wherein the disposable part further comprises an agent outlet communicating with the agent reservoir and the transdermal patch.
9. The system of item 8, wherein the agent outlet is configured to provide the bioactive agent dissolved in the solvent to a space between the transdermal patch and the vapor permeable membrane.
10. The system of item 1, wherein the agent reservoir comprises a piston movably disposed in a chamber.
11. The system of item 10, further comprising a spring extending between the agent reservoir piston and a surface to pressurize the agent reservoir when the spring is compressed and the agent reservoir contains a quantity of bioactive agent solution.
12. The system of item 10, further comprising a bolus chamber communicating with the agent reservoir and an agent outlet, the bolus chamber comprising a piston movably disposed in a chamber, the volume of the bolus chamber being less than the volume of the agent reservoir.
13. The system of item 12, further comprising a valve having a first position communicating the agent reservoir with the bolus chamber and a second position communicating the bolus chamber with the agent outlet.
14. The system of item 13, wherein the reusable part comprises a valve driver, the control electronics being adapted to control the valve driver to actuate the valve to deliver bioactive agent solution from the agent reservoir to the bolus chamber and from the bolus chamber to the agent outlet.
15. The system of item 10, further comprising a spring extending between the bolus chamber piston and a surface to pressurize the bolus chamber when the spring is compressed and the bolus chamber contains a quantity of bioactive agent solution.
16. The system of item 1, further comprising a latch adapted to removably attach the disposable part to the reusable part.
17. The system of item 16, wherein the latch is positioned on one side of the system and a rail is positioned along a second side of the system, the disposable part and the reusable part configured to slide relative to one another along the rail until the latch is activated to attach the disposable part to the reusable part.
18. The system of item 1, further comprising a connection detector adapted to detect a connection between the disposable part and the reusable part.
19. The system of item 18, wherein the connection detector comprises a magnet.
20. The system of item 19, wherein the magnet is disposed in the disposable part.
21. The system of item 19, wherein the magnet is disposed in the reusable part.
22. The system of item 19, wherein the connection detector further comprises a magnetoresistive switch.
23. A method of delivering a bioactive agent, the method comprising:
   connecting a reusable part of a delivery system to a disposable part of the delivery system to form a flow path between the reusable part and the disposable part;
   delivering the bioactive agent dissolved in a solvent from a reservoir of the disposable part to a transdermal membrane of the disposable part; and
   allowing the solvent to evaporate and flow from the transdermal membrane through a gas permeable membrane and along the flow path to ambient air around the delivery system.
24. The method of item 23, further comprising applying the transdermal membrane to skin of a user.
25. The method of item 24, further comprising delivering the bioactive agent to the skin.
26. The method of item 23, wherein the delivering step comprises controlling with the reusable part movement of the bioactive agent dissolved in the solvent from the reservoir to the transdermal membrane.
27. The method of item 26, wherein the controlling step comprises actuating a valve.
28. The method of item 23, further comprising detecting a connection between the disposable part and the reusable part.
29. The method of item 28, wherein the detecting step comprises sensing a magnetic field.

## Claims

1. A two-part bioactive agent delivery system, the system comprising:
a disposable part comprising:
a chassis;
an agent reservoir;
a transdermal patch communicating with the agent reservoir and adapted to transdermally deliver the bioactive agent to a user, the transdermal patch having a bottom surface adapted to contact skin of the user and a top surface opposite the bottom surface; and
a gas permeable membrane disposed over the top surface of the transdermal patch;
a reusable part comprising a power source and control electronics, the control electronics being adapted to deliver bioactive agent dissolved in a solvent from the agent reservoir to the transdermal patch;
a solvent removal element comprising a gap disposed between the disposable part and the reusable part to create a flow path for gaseous solvent to flow from the gas permeable membrane to ambient air around the bioactive agent delivery system; and
a vent through the chassis configured to expose the gas-permeable membrane underneath, said vent being positioned on a central portion of the chassis.

2. The system of claim 1, wherein the vent is configured to expose 5-30% of a surface of the gas permeable membrane.

3. The system of claim 1, wherein the gap is maintained with a spacer disposed between the disposable part and the reusable part.

4. The system of claim 1, wherein the disposable part comprises the vent.

5. The system of claim 1, further comprising a diagonal strut extending through the vent configured to maintain strength of the chassis.

6. The system of claim 1, further comprising a plurality of vents, and wherein diagonal struts extend between the plurality of vents.

7. The system of claim 1, wherein one or more spacers are positioned around the vent.

8. The system of claim 1, further comprising at least one drainage port formed in an exterior surface of the reusable part or the disposable part.

9. The system of claim 1, wherein the disposable part further comprises an agent outlet communicating with the agent reservoir and the transdermal patch.

10. The system of claim 9, wherein the agent outlet is configured to provide the bioactive agent dissolved in the solvent to a space between the transdermal patch and the gas permeable membrane.

11. The system of claim 1, wherein the agent reservoir comprises a piston movably disposed in a chamber.

12. The system of claim 11, further comprising a spring extending between the piston and a surface to pressurize the agent reservoir when the spring is compressed and the agent reservoir contains a quantity of bioactive agent solution.

13. The system of claim 11, further comprising a bolus chamber communicating with the agent reservoir and an agent outlet, the bolus chamber comprising a second piston movably disposed in a chamber, the volume of the bolus chamber being less than the volume of the agent reservoir.

14. The system of claim 13, further comprising a valve having a first position communicating the agent reservoir with the bolus chamber and a second position communicating the bolus chamber with the agent outlet.

15. The system of claim 14, wherein the reusable part comprises a valve driver, the control electronics being adapted to control the valve driver to actuate the valve to deliver bioactive agent solution from the agent reservoir to the bolus chamber and from the bolus chamber to the agent outlet.
